(19) 

**Europäisches Patentamt**
**European Patent Office**
**Office européen des brevets**

(11) **EP 4 678 761 A1**

(12) **EUROPEAN PATENT APPLICATION**

(43) Date of publication:
**14.01.2026  Bulletin 2026/03**

(21) Application number: **24306182.7**

(22) Date of filing: **12.07.2024**

(51) International Patent Classification (IPC):
*C12P 19/04* (2006.01)      *C12P 19/14* (2006.01)

(52) Cooperative Patent Classification (CPC):
**C12P 19/14; C12P 19/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **ROQUETTE FRERES**
**62136 Lestrem (FR)**

(72) Inventors:
• **BOIT, Baptiste**
 **59253 La Gorgue (FR)**

• **GRAS, Ludivine**
 **62136 Lestrem (FR)**
• **CARRE, Yoann**
 **62400 Béthune (FR)**
• **DESQUIEN, Lise**
 **59251  Allennes-les-Marais (FR)**
• **TOURSEL, Béatrice**
 **62920 Gonnehem (FR)**
• **DEHAY, Frédérick**
 **62840 Laventie (FR)**

(74) Representative: **Plasseraud IP**
**104 Rue de Richelieu
CS92104
75080 Paris Cedex 02 (FR)**

(54) **METHOD OF PRODUCING HIGH AND LOW MOLECULAR WEIGHT PECTIC SACCHARIDES FROM POTATO**

(57)    The invention relates to a method of producing high and low molecular weight pectic saccharides that are enriched in HG and RG-I, said method comprising the steps providing potato pulp as pectin-rich substrate, subjecting the pectin-rich substrate to enzymatic treatment, said enzymatic treatment comprising the use of one pectinase having an endo-polygalacturonase (EC 3.2.1.15) activity, subjecting the hydrolyzed pectic polysaccharides to microfiltration using microfiltration membranes having a molecular cut-off of 0.45 and 0.8 $\mu$m, recovering the microfiltration retentate, subjecting the microfiltration permeate to nanofiltration using a membrane having a molecular weight cut-off in the range of 150 - 300 Da, recovering the nanofiltration retentate.

**EP 4 678 761 A1**

**Description**

TECHNICAL FIELD OF THE INVENTION

The present invention relates to a method of producing high and low molecular weight pectic saccharides enriched in rhamnogalacturonan-I (RG-I) and homogalacturonan (HG) from potato pulp.

More particularly, the invention relates to a method of producing such pectic saccharides by enzymatic hydrolysis of pectin-rich substrate that has been obtained from potato material, followed by multi-step of filtration, more specifically microfiltration and nanofiltration, and recovery of the high and low molecular weight pectic saccharides.

BACKGROUND OF THE INVENTION

Pectin is a complex mixture of colloidal polysaccharides found in the primary cell walls of both monocotyledons and dicotyledons.

The main polysaccharide structures are homogalacturonan (HG), rhamnogalacturonan-I (RG-I), and substituted galacturonans.

HG is a linear chain of 1,4- linked $\alpha$-D-gatactopyranosyturonic acid (GalA) residues in which some of the carboxyl groups are methyl esterified.

Rhamnogalacturonan (RG-1) has been called the "hairy region" of pectin, and is a family of pectic polysaccharides that contain a backbone of the repeating disaccharide [->4)-$\alpha$-D-GalA-(I->2)-$\alpha$-I-Rha-(I->].

20-80% of the rhamnosyl (Rha) residues are, depending on the plant source and method of isolation, substituted at C-4 with neutral and acidic oligosaccharide side chains.

The predominant side chains contain linear and branched $\alpha$-1-arabinofuranosyl (Ara), and/or $\beta$-D-galactopyranosyl (Gal) residues although their relative proportions and chain lengths may differ depending on the plant source.

Some of the side chains may be terminated with $\alpha$-L- Fucopyranose (Fuc), $\beta$-D-Glucuronic acid (GlcA), and 4-O-methyl $\beta$-D-GlcA residues.

The backbone GalA is not typically substituted with oligosaccharides, and the backbone GalA residues are O-acetylated in rhamnogalacturonans from many plants.

Rhamnogalacturonan-I (RG-I) has been previously isolated from many plants and vegetables including potato.

Isolated Rhamnogalacturonan-I is typically a polysaccharide with a molecular weight in the 100,000 Da - 1,000,000 Da size range.

When pure, RG-1 is typically soluble in water with low intrinsic viscosity. It has been discussed to have mitogenic, immunostimulation and antitumor activity.

As the structure, size and composition of RG-1 determines its bioactivity, small changes in structure may confer large changes in activity.

Potato pulp is as a major low-value product generated primarily upon starch production or potato processing. Despite its interesting phytochemical composition, potato pulp was mainly used as cattle feed.

Major components of potato pulp are cell wall polysaccharides, which include cellulose, hemicellulose and pectic polysaccharides.

Pectic polysaccharides (56%) being the major components of potato cell wall have low economic value because of its low gelling properties associated with its high content of neutral sugars.

Indeed, unlike pectic polysaccharides from other sources, such as citrus and apple, potato pectin consists of high proportion of rhamnogalacturonan I (RG I) (72%) and smaller amount of homogalacturonan (HG).

Second distinguished structural property of potato pectin arise from the high proportion of neutral $\beta$-linked galactan side chains (representing 46% of the total pectin) linked to RG I backbone; while RG I originating from currently commercial sources of pectin are mostly substituted with arabinan side chains.

In spite of the interesting structural properties of pectic polysaccharides and their low cost, so far, they have not been often used as sources for the production of bioactive molecules.

Improved selective extraction and purification technologies are required if products are to be available on a large enough scale for health applications.

Without being exhaustive, the following literature lists various processes for isolating pectic polysaccharides form potato :

WO 2015/192247 relates to a process of isolating non-digestible oligosaccharides from potato pulp by extracting rhamnogalacturonan content from the potato pulp, digesting the extracted rhamnogalacturonan content with a multi-enzymatic mixture to yield the non- digestible oligosaccharides from the extracted rhamnogalacturonan content; and isolating the non-digestible oligosaccharides. Also are described pharmaceutical, nutraceutical and food applications for these protein-enriched preparations.

WO 2016/132130 describes a process for the isolation of modified polysaccharide product from potato, in particular a polysaccharide product that can be used as an immunomodulatory.

WO 2000/043424, WO 2001/096405 and WO 2007/119366 relates to process for producing pectin involving the step of extracting root crops with hot water under weakly acidic conditions of pH, pectin having good qualities which is obtained by extracting from root crops; and acidic protein foods with the use of the same which are stable in the acidic pH region of the isoelectric point of protein or higher, and finally an extraction process of a pectin having excellent gelling effect from a potato starch pulp with high efficiency (with a chelating agent at a temperature of 90 to 125°C inclusive under condition where the pH value given after the completion of the extraction is adjusted to 5.5 to 11), and to produce a gelatinous food by using the pectin as a gelling agent.

WO 2005/003178 relates to a method for treating pectin-containing plant materials in a manner to obtain fibre-containing pectin products, and subsequently pectin products, having a high molecular weight of the pectin polymer and a homogeneous distribution of the de-esterified sites in the pectin polymer and thereby providing products having improved gel forming and/or viscous giving properties.

WO 2013/148282 provides nutritional supplement compositions. For example, nutritional supplement compositions containing a potato polysaccharide preparation, methods for obtaining potato polysaccharide preparations, methods for making nutritional supplement compositions containing a potato polysaccharide preparation, and methods for increasing or decreasing expression of polypeptides involved with mitochondria activity or function are provided.

So, it may especially be most interesting to have new pectic saccharides enriched in rhamnogalacturonan-I (RG-I) and homogalacturonan (HG) to propose.

SUMMARY OF THE INVENTION

To this end, the applicant company has pursued much laborious study and research which has enabled it to develop products which meet these requirements.

The applicant company have shown that high and low molecular weight pectic saccharides enriched in RGI and HG with biological functionalities can be obtained by a method comprising:

- subjecting a pectin-rich substrate that has been obtained from potato pulp to one pectinase having endo-polygalacturonase (EC 3.2.1.15) activity,
- subjecting the hydrolyzed pectic polysaccharides such obtained to microfiltration and nanofiltration and
- recovering the microfiltration and nanofiltration fractions.

More particularly, the present invention relates to a method of producing high and low molecular weight pectic saccharides that are enriched in HG and RG-I, said method comprising the steps of:

- providing potato pulp as pectin-rich substrate,
- subjecting the pectin-rich substrate to enzymatic treatment, said enzymatic treatment comprising the use of one pectinase having an endo-polygalacturonase (EC 3.2.1.15) activity,
- subjecting the hydrolyzed pectic polysaccharides to microfiltration using microfiltration membranes having a molecular cut-off of between 0.45 and 0.8 $\mu$m,
- recovering the microfiltration retentate,
- subjecting the microfiltration permeate to nanofiltration using a membrane having a molecular weight cut-off in the range of 150 - 300 Da,
- recovering the nanofiltration retentate.

The applicant company has first found that contrary to what is taught in the prior art, it is not useful to implement a cocktail of enzymes to achieve the supply of a such new pectic saccharides.

The hydrolyzed pectic polysaccharides present:

- a molecular weight distribution for the fractions:

  ○ lower than 1500 Da, of between 10 and 25 %, preferably between 16 and 19 %,
  ○ from 1500 Da to 100 kDa, of between 45 and 65 %, preferably between 52 and 58 %, and
  ○ higher than 100 kDa between 20 and 35%, preferably between 26 and 29%,

- between 20 and 30 % in mol%, preferably between 22 and 27 % in mol% of HG,
- between 70 and 75 % in mol% preferably between 71 and 74 % in mol% of RG-I having a GalA to Rha ratio between 6.5 and 7.5 mol/mol, preferably between 6.6 and 7.4 mol/mol,

and a (Gal plus Ara) to GalA ratio between 1.8 to 3.0 mol/mol, preferably between 2.0 and 2.6 mol/mol, - a methylation degree between 40 and 60 % in mol%, preferably between 44 and 57 % in mol% and an acetylation degree between 45 and 50 % in mol%, preferably between 47 and 49 % in mol%.

The hydrolyzed pectic polysaccharides is also characterized by its RG-I side chains having:

- a (Gal plus Ara) to Rha ratio between 10 and 20 mol/mol, preferably between 13 to 19 mol/mol,
- a Gal to Rha ratio between 8 and 20 mol/mol, more preferably between 10 and 16 mol/mol,
- an Ara to Rha ratio between 2.5 and 3.5 mol/mol, more preferably between 2.8 and 3.4 mol/mol,
- a GalA to Gal ratio between 0.3 and 1.0 mol/mol, preferably between 0.5 and 0.6 mol/mol.

The applicant company have also found that enzymatic hydrolysis of the pectic polysaccharides using only one aforementioned pectinase followed by microfiltration, yields a high molecular weight pectic saccharide that exhibits biological functionalities.

So, the present invention relates to the microfiltration retentate containing high molecular weight pectic saccharide having :

- a molecular weight distribution for the fractions:

    ○ lower than 1500 Da, of between 5 and 15 %, preferably between 7 and 12 %,
    ○ from 1500 Da to 100 kDa, of between 40 and 65 %, preferably between 47 and 56 %, and
    ○ higher than 100 kDa, of between 25 and 50%, preferably between 32 and 46%,

- between 15 and 28 % in mol%, preferably between 16 and 25 % in mol% of HG,
- between 70 and 85 % in mol%, preferably between 73 and 83 % in mol% of RG-I having a GalA to Rha ratio between 4.0 and 6.0 mol/mol, preferably between 4.1 and 5.5 mol/mol, and a (Gal plus Ara) to Gal A ratio between 1.5 to 4.0 mol/mol, preferably between 1.9 and 3.5 mol/mol,
- a methylation degree between 15 and 40 % in mol% preferably between 17 and 39 % in mol% and an acetylation degree between 50 and 95 % in mol%, preferably between 51 and 90 % in mol%.

The terminology "degree of acetylation" refers to the number of acetyl residues per galacturonic acid residue, expressed as a molar percentage.

The terminology "degree of methylation" refers to the number of methyl residues per galacturonic acid residue, expressed as a molar percentage.

The concentration of different polysaccharides and their monosaccharide composition can be determined by analytical techniques known to the skilled person.

All percentages mentioned herein, unless otherwise stated, refer to the percentage in % by dry weight.

The RG-I side chains of the high molecular weight pectic saccharides of the invention are characterized by:

- a (Gal plus Ara) to Rha ratio between 9 to 15 mol/mol, preferably between 10 and 14 mol/mol,
- a Galto Rha ratio between 5 and 13 mol/mol, more preferably between 7 and 11 mol/mol,
- an Ara to Rha ratio between 2.0 and 4.0 mol/mol, more preferably between 2.4 and 3.5 mol/mol,
- a GalA to Gal ratio between 0.2 and 1.0 mol/mol, preferably between 0.4 and 0.7 mol/mol.

The high molecular weight pectic saccharides of the invention may be also characterized by their total sugar composition and their free sugars compositions, as will be exemplified below.

As it will be exemplified below, its total sugar composition is mainly :

- Ara: between 8 and 14 %w/w, preferably 9 and 12 %w/w,
- Rha: between 3.0 and 5.0 %w/w, preferably between 3.7 and 4.5 %w/w,
- Gal: between 32 and 48 %w/w, more preferably between 36 and 44 %w/w,
- GalA: between 16 and 30 %w/w, more preferably between 18 and 28 %w/w.

Then, the following nanofiltration step that eliminate notably DP1 - DP2 (DP for Degree of Polymerization) of the microfiltration permeate yields a low molecular weight pectic saccharide that exhibits other biological functionalities.

So, the present invention relates to the nanofiltration permeate containing low molecular weight pectic saccharide having :

- a molecular weight distribution for the fractions:

- lower than 1500 Da between 10 and 30%, preferably between 17 and 25%
- from 1500 Da to 100 kDa between 60 and 80%, preferably between 65 and 79%, and
- higher than 100 kDa between 0 and 25%, preferably between 1 and 17%,

- between 20 and 35 % in mol%, preferably between 22 and 33 % in mol% of HG
- between 65 and 75 % in mol%, preferably between 66 and 74 % in mol% of RG-I having a GalA to Rha ratio between 5 and 30 mol/mol, preferably between 7 and 26 mol/mol, and a (Gal plus Ara) to GalA ratio between 1.5 and 3.0 mol/mol, preferably between 1.8 and 2.6 mol/mol.
- a methylation degree between 20 and 35 % in mol%, preferably between 23 and 44 % in mol% and an acetylation degree between 10 and 40 % in mol%, preferably between 11 and 38 % in mol%.

The RG-I side chains of the low molecular weight pectic saccharides of the invention are characterized by:

- a (Gal plus Ara) to Rha ratio between 15 and 60 mol/mol, preferably between 18 and 55 mol/mol,
- a Gal to Rha ratio between 10 and 50 mol/mol, more preferably between 15 and 48 mol/mol,
- an Ara to Rha ratio between 2.8 and 7.0 mol/mol, more preferably between 3.1 and 6.4 mol/mol,
- a GalA to Gal ratio between 0.3 and 0.7 mol/mol, preferably between 0.5 and 0.6 mol/mol.

The low molecular weight pectic saccharides of the invention may be also characterized by their total sugar composition and their free sugars compositions, as will be exemplified below.

As it will be exemplified below, its total sugar composition is mainly :

- Ara: between 4.0 and 10.0 %w/w, preferably 4.8 and 8.1 %w/w,
- Rha: between 0.5 and 3.0 %w/w, preferably 0.9 and 2.8 %w/w,
- Gal: between 44 and 52 %w/w, more preferably between 46 and 50 %w/w,
- GalA: between 22 and 36 %w/w, more preferably between 26 and 34 %w/w.

The present invention further relates to the high and low molecular weight pectic saccharides obtained by the present method and to a process of preparing a product selected from a nutritional formulation, a food product, a dietary supplement, a beverage or a pharmaceutical product, said process comprising addition of the aforementioned pectic saccharide.

## DETAILED DESCRIPTION OF THE INVENTION

Accordingly, a first aspect of the invention relates to a method of producing high and low molecular weight pectic saccharides that are enriched in HG and RG-I, said method comprising the steps of:

- providing potato pulp as pectin-rich substrate,
- subjecting the pectin-rich substrate to enzymatic treatment, said enzymatic treatment comprising the use of one pectinase having an endo-polygalacturonase (EC 3.2.1.15) activity,
- subjecting the hydrolyzed pectic polysaccharides to microfiltration using a microfiltration membrane having a molecular cut-off of 0.45 and 0.8 μm,
- recovering the microfiltration retentate,
- subjecting the microfiltration permeate to nanofiltration using a membrane having a molecular weight cut-off in the range of 150 - 300 Da,
- recovering the nanofiltration retentate.

The first step of the process following the present invention consists in providing potato pulp as pectin-rich substrate.

Potato pulp is a major waste product of the potato-processing industry. Approximately 0.75 kg of potato pulp is generated to produce 1.0 kg of starch.

On a dry weight basis, the non-starch components of potato pulp include fibers (88.3%), proteins (6.3%), ashes (5.0%) and fat (0.4%).

The moisture (8.7 - 87.0%w/w) and starch content (13.1 - 37.0% by dry weight) of potato pulp varies depending on the starch extraction method and any further drying process.

The pectin-rich substrate used in the present method preferably contains at least 25 % by dry weight, more preferably at least 28 % by dry weight (the pectin content is determined by summing the contents of Gal, Ara, Rha and GalA sugars, quantified using the analytical methods described below).

EP 4 678 761 A1

The second step of the process following the present invention consists in subjecting the pectin-rich substrate to enzymatic treatment.

The enzymatic treatment employs one specific pectinase, more particularly an endopolygalacturonase (EC 3.2.1.15).

The pure galacturonase may be the ISOZM PG200 form MCN Biotech or the Polygalacturonase PF from the company Soufflet Biotechnologies.

The polygalacturonase enzyme activity must be known in advance and quantified by a method known to the person skilled in the art, such as the codified method used in oenology (COEI-1-ACTPGA).

The result are expressed in the unit uPG in relation to the mass quantity of commercial solution (uPG/g) as described in https://www.oiv.int/index.php/standards/international-oenoloqical-codex/part-i-monographs/enzymes/poly galacturonase-activity

For the treatment, the enzyme dose is in the range of 20 to 55 uPG by grams of pulp on dry matter, preferably of 25 to 50 uPG by grams of pulp on dry matter.

The pectin-rich substrate is subjected to one enzymatic treatment (enzyme dose in the range of 20 to 55 uPG by grams of pulp on dry matter, preferably of 25 to 50 uPG by grams of pulp on dry matter) in the form of an aqueous liquid containing the pectin-rich substrate and having a dry matter content of 2 to 20 % In weight, preferably of 5 to 15 % in weight.

The enzymatic treatment of the method according to the invention is preferably carried out at a pH in the range of 2.5 to 6.0, preferably in the range of 3.5 to 5.5.

The enzymatic treatment of the pectin-rich substrate with one or more pectinases is preferably carried out at a temperature in the range of 40° to 55°C, more preferably of 50°C.

The duration of the enzymatic treatment preferably is between 2 to 3 hours.

According to a particular mode of realization of the invention, a liquid/solid separation step is carried out to recover the hydrolyzed pectic polysaccharides fraction.

This operation can be carried out using various technologies, with a potential combination of these technologies (to maximize recovery yields), for example: decanter centrifuge, disc stack centrifuge, belt press, hydraulic filter press, rotary vacuum filter.

A thermal treatment is also performed, with the objective to inactivate the enzyme.

The operation is carried out by applying a defined temperature/treatment time scale, e.g. 70°C for 2 h, or 90°C for 30min.

This operation can also be carried out using a high-pressure thermal process, well known by the man skilled in the Art.

The hydrolyzed pectic polysaccharides present :

- a molecular weight distribution for the fractions:

  ○ lower than 1500 Da, of between 10 and 25 %, preferably between 16 and 19 %,
  ○ from 1500 Da to 100 kDa, of between 45 and 65 %, preferably between 52 and 58 %, and
  ○ higher than 100 kDa between 20 and 35%, preferably between 26 and 29%,

- between 20 and 30 % in mol%, preferably between 22 and 27 % in mol% of HG,
- between 70 and 75 % in mol% preferably between 71 and 74 % in mol% of RG-I having a GalA to Rha ratio between 6.5 and 7.5 mol/mol, preferably between 6.6 and 7.4 mol/mol, and a (Gal plus Ara) to GalA ratio between 1.8 to 3.0 mol/mol, preferably between 2.0 and 2.6 mol/mol,
- a methylation degree between 40 and 60 % in mol%, preferably between 44 and 57 % in mol% and an acetylation degree between 45 and 50 % in mol%, preferably between 47 and 49 % in mol%.

The HG domains do not contain any sidechains. The carboxyl groups of galacturonic acid residues within the backbone of HG domains may be esterified. Esterified galacturonic acid may occur in the form of the methyl ester or acetyl ester.

The backbone of RG-I contains side chains that are composed of Galactose (Gal), Arabinose (Ara), Rhamnose (Rha) and GalA (Galacturonic Acid), here in the following proportion:

- a (Gal plus Ara) to Rha ratio between 10 and 20 mol/mol, preferably between 13 and 19 mol/mol,
- a Gal to Rha ratio between 8 and 20 mol/mol, more preferably between 10 and 16 mol/mol,
- an Ara to Rha ratio between 2.5 and 3.5 mol/mol, more preferably between 2.8 and 3.4 mol/mol,
- a GalA to Gal ratio between 0.3 and 1.0 mol/mol, preferably between 0.5 and 0.6 mol/mol.

The hydrolyzed pectic polysaccharides such obtained may be also characterized by their total sugar composition and their free sugars compositions.

As it will be exemplified below, its total sugar composition is mainly :

- Ara: between 7.0 and 9.0 %w/w, preferably 7.5 and 8.4 %w/w,
- Rha: between 1.5 and 4.0 %w/w, preferably between 2.1 and 3.2 %w/w,
- Gal: between 35 and 55 %w/w, more preferably between 37 and 50 %w/w,
- GalA: between 20 and 30 %w/w, more preferably between 22 and 28 %w/w.

The third step of the process following the invention consists in subjecting the hydrolyzed pectic polysaccharides to microfiltration.

The microfiltration treatment employs using a membrane having a molecular cut-off of between 0.45 and 0.8 μm, chosen in the group consisting in ceramic tubular membrane, ceramic disc membrane, organic spiral wound membrane, flat sheet membrane, hollow fiber membrane chosen alone or in combination. Thus, microfiltration can be carried out in one or two successive stages using membranes with the different cut-off values.

The fourth step of the process following the invention consists in recovering the microfiltration retentate, that could be optionally evaporated and dried by any techniques known by the man skilled in the Art.

The recovered microfiltration retentate contains the high molecular weight pectic saccharide of the invention.

This retentate may suitably be concentrated and then dried using one or more drying techniques selected from spray drying, freeze drying, air drying, roller drying, flatbed drying, belt drying and drum drying.

The high molecular weight pectic saccharide such obtained has:

- a molecular weight distribution for the fractions:

    ◦ lower than 1500 Da, of between 5 and 15 %, preferably between 7 and 12 %,
    ◦ from 1500 Da to 100 kDa, of between 40 and 65 %, preferably between 47 and 56 %, and
    ◦ higher than 100 kDa, of between 25 and 50%, preferably between 32 and 46%,

- between 15 and 28 % in mol%, preferably between 16 and 25 % in mol% of HG,
- between 70 and 85 % in mol%, preferably between 73 and 83 % in mol% of RG-I having a GalA to Rha ratio between 4.0 and 6.0 mol/mol, preferably between 4.1 and 5.5 mol/mol, and a (Gal plus Ara) to Gal A ratio between 1.5 to 4.0 mol/mol, preferably between 1.9 and 3.5 mol/mol,
- a methylation degree between 15 and 40 % in mol% preferably between 17 and 39 % in mol% and an acetylation degree between 50 and 95 % in mol%, preferably between 51 and 90 % in mol%.

The RG-I side chains of the high molecular weight pectic saccharides of the invention are characterized by:

- a (Gal plus Ara) to Rha ratio between 9 and 15 mol/mol, preferably between 10 and 14 mol/mol,
- a Gal to Rha ratio between 5 and 13 mol/mol, more preferably between 7 and 11 mol/mol,
- an Ara to Rha ratio between 2.0 and 4.0 mol/mol, more preferably between 2.4 and 3.5 mol/mol,
- a GalA to Gal ratio between 0.2 and 1.0 mol/mol, preferably between 0.4 and 0.7 mol/mol.

The high molecular weight pectic saccharides of the invention may be also characterized by their total sugar composition and their free sugars compositions, as will be exemplified below.

As it will be exemplified below, its total sugar composition is mainly :

- Ara: between 8 and 14 %w/w, preferably 9 and 12 %w/w,
- Rha: between 3.0 and 5.0 %w/w, preferably between 3.7 and 4.5 %w/w,
- Gal: between 32 and 48 %w/w, more preferably between 36 and 44 %w/w,
- GalA: between 16 and 30 %w/w, more preferably between 18 and 28 %w/w.

The fifth step of the process following the invention consists in subjecting the final microfiltration permeate to nanofiltration using a membrane having a molecular weight cut-off in the range of 150 - 300 Da.

The recovered nanofiltration retentate contains the low molecular weight pectic saccharide of the invention.

This retentate may suitably be concentrated and then dried using one or more drying techniques selected from spray drying, freeze drying, air drying, roller drying, flatbed drying, belt drying and drum drying.

The low molecular weight pectic saccharide such obtained has:

- a molecular weight distribution for the fractions:

∘ lower than 1500 Da between 10 and 30%, preferably between 17 and 25%
∘ from 1500 Da to 100 kDa between 60 and 80%, preferably between 65 and 79%, and
∘ higher than 100 kDa between 0 and 25%, preferably between 1 and 17%,

- between 20 to 35 % in mol%, preferably between 22 to 33 % in mol% of HG
- between 65 to 75 % in mol%, preferably between 66 to 74 % in mol% of RG-I having a GalA to Rha ratio between 5 to 30 mol/mol, preferably between 7 to 26 mol/mol, and a (Gal plus Ara) to GalA ratio between 1.5 to 3.0 mol/mol, preferably between 1.8 to 2.6 mol/mol.
- a methylation degree between 20 and 35 % in mol%, preferably between 23 to 44 % in mol% and an acetylation degree between 10 to 40 % in mol%, preferably between 11 to 38 % in mol%.

The RG-I side chains of the low molecular weight pectic saccharides of the invention are characterized by:

- a (Gal plus Ara) to Rha ratio between 15 and 60 mol/mol, preferably between 18 and 55 mol/mol,
- a Gal to Rha ratio between 10 and 50 mol/mol, more preferably between 15 and 48 mol/mol,
- an Ara to Rha ratio between 2.8 and 7.0 mol/mol, more preferably between 3.1 and 6.4 mol/mol,
- a GalA to Gal ratio between 0.3 and 0.7 mol/mol, preferably between 0.5 and 0.6 mol/mol.

The low molecular weight pectic saccharides of the invention may be also characterized by their total sugar composition and their free sugars compositions, as will be exemplified below.
As it will be exemplified below, its total sugar composition is mainly :

- Ara: between 4.0 and 10.0 %w/w, preferably 4.8 and 8.1 %w/w,
- Rha: between 0.5 and 3.0 %w/w, preferably 0.9 and 2.8 %w/w,
- Gal: between 44 and 52 %w/w, more preferably between 46 and 50 %w/w,
- GalA: between 22 and 36 %w/w, more preferably between 26 and 34 %w/w.

A final subject of the present invention relates to the use of the high or low molecular weight pectic saccharides according to the invention, or able to be obtained according to the method defined above, in nutritional formulation, food product, dietary supplement, beverage or pharmaceutical product.
The examples which follow make it possible to better understand the present invention, without however limiting the scope thereof.

## EXAMPLES

ANALYTICAL METHODS

### *Molecular mass distribution:*

Throughout the present application, the Mw, Mn, and PDI were determined by high pressure size-exclusion chromatography (HPSEC).
This method permits to separate molecules in solution by their size, which correlates to their molecular weight and are compared to Pullulan standards with known molecular weight. Pullulan standards (Grade P-5, P-10, P-20, P-50, P-100, P-200, P-400 and P-800) are purchased from Showa Denko K.K. (Japan); glycerin, glucose, maltose, maltotriose, maltotetraose, maltopentaose, maltohexaose and maltoheptaose are purchased from Sigma Aldrich. 0.5 % standard solutions are prepared by dissolving 50 mg of each standard and 50 mg of glycerin (output marker) in 10.0 mL of eluent solvent and then filtered through a 0.45 micron filter. Samples of the carbohydrate compositions are first de-ashed with 1.0 g LEWATIT S4228 and PUROLITE C 150 MBH mixed resins and then diluted with eluent solvent to about 2.5 %, 0.25 % of glycerine (output marker) is added, followed by filtration through a 0.45 micron filter.
100 μL of either the standard solutions or sample solutions are injected into a HPLC system equipped with a refractive index detector and 3 SHODEX SEC columns (OHpak SB-805 HQ, SB-803 HQ, SB-802 HQ connected in series). The chromatography is performed with following parameters:

- Injecting volume: 100 μL
- Flowrate: 0.5 mL/min
- Column temperature: 35°C
- Eluent: sodium nitrate 0.1M + sodium azide 0.02 % filtered on a 0.02 micron filter
- Eluting time: 80 min

The resulting chromatograms are analyzed offline using Empower software. The liberated mono-, di-, oligo- and polysaccharides size is determined according to the eluting time comparing to glycerin, glucose, maltose, maltotriose, maltotetraose, maltopentaose, maltohexaose, maltoheptaose and Pullulans standard eluting time.

From the GPC distribution table, it is determined the cumulative percentage of saccharides having these following range of molecular weights:

- Fraction lower than 1500 Da
- Fraction from 1500 Da to 100 kDa
- Fraction higher than 100 kDa.

***Total neutral sugars composition:***

The sugars are determined by gas chromatography in the form of silylated methoxime derivatives and then quantified using the internal calibration method. Galactitol is used as the internal standard.

The analysis is carried out using the following equipment:

- A gas chromatograph (type VARIAN 3400) equipped with:

  ○ A split-splitless injector fitted with a split liner,
  ○ A flame ionisation detector,
  ○ An integrator-recorder or any computer system for processing the detector signal.

- A DB-1 GC capillary column

  ○ Length: 40 metres
  ○ Internal diameter: 0.18 millimetres
  ○ Film thickness: 0.40 microns

Chromatographic conditions:

- Column temperature: 100°C to 250°C at 3°C/min, then 250 to 325°C at 15°C/min. Hold for 16 minutes.
- Helium pressure: 40 psi (in constant flow mode)
- Injection mode: split
- Split flow rate: 100 mL/min
- Injector temperature (split/splitless): 325°C
- FID detector temperature: 330°C
- Injected volume: 1 μL

According to the following protocol:

1) Internal standard solution
Prepare a solution of galactitol at approximately precisely 5 mg/mL in osmosis water.

2) Hydrochloric hydrolysis
In duplicate: in a 15 ml hydrolysis tube, weigh approximately 50 to 500 mg of sample precisely, add 2 mL of the internal standard solution, add 3 mL of water and 5 mL of the 4N HCl solution. Stir for 1 min on a vortex mixer. Place the tube in a thermostated dry bath regulated at 100°C for 1 hour, vortexing from time to time.

3) Demineralisation and concentration
After cooling, transfer the entire hydrolysis to a 50 ml beaker. Add 6 to 8 g of a 50/50 mixture of AG 4-X4 anion resin and AG 50W-X8 cation resin. Leave under magnetic stirring for 5 minutes. Filter the contents of the beaker and recover the juice in the rinsed hydrolysis tube. Repeat the demineralization step until a neutral pH is obtained.

4) Preparing the derivative
Place approximately 1 mL of the demineralised hydrolysis in a 2 mL well, add approximately 1 mL of pyridine and evaporate to dryness under a stream of nitrogen. Take up with 20 mg methoxylamine hydrochloride and 1 mL pyridine. Leave for 40 min at 70°C. Add 0.5 mL BSTFA and leave for 30 min at 70°C. Cool to room temperature before injecting.

5) Calculation

Read the area of the peaks corresponding to each sugar and the internal standard.

The total sugar content expressed in g per 100 g of raw product is given by the equation:

$$Sugar\ content\ i\ (\%/raw) = \frac{S_i}{S_e} \times \frac{P_e}{P} \times \frac{100}{K_i}$$

With:

$S_i$ = area of the peak(s) of sugar i
$S_e$ = Area of the internal standard peak
$P_e$ = weight of internal standard introduced into the beaker (in mg) (corrected weight - Purity/Dry matter).
$P$ = weight of sample introduced into the beaker (in mg)
$K_i$ = response coefficient of sugar i

6) System calibration:

- Standard solution:
  Use the classic 'methoximated sugars' solution containing each of the sugars at approximately precisely 100mg of each sugar in 200ml of water.
- Determination of response coefficients:
  In a 15 ml hydrolysis tube, add 5 mL of the 'methoximated sugars' solution, 2 mL of the internal standard solution, add 3 mL of water and 5 mL of the 4N HCl solution. Stir for 1 min with a vortex mixer. Place the tube in a thermostated dry bath regulated at 100°C for 1 hour, vortexing from time to time. Then, as for the samples, proceed with demineralization and derivatization as in 3 and 4.

Record the area of the peak(s) corresponding to each sugar and the internal standard. The response coefficient $K_i$ for each species i is given by the following equation:

$$K_i = \frac{S_i}{S_e} \times \frac{P_e}{P_i}$$

With :

$S_i$ = area of the sugar i peak(s)
$S_e$ = Surface area of the internal standard peak
$P_e$ = weight of internal standard introduced into the hydrolysis tube (in mg) (corrected weight - Purity/Dry matter)
$P_i$ = weight of sugar i introduced into the hydrolysis tube (in mg)

*Free neutral sugars composition:*

The sugars are determined by gas chromatography in the form of silylated methoxime derivatives and then quantified using the internal calibration method. Methylalpha-D-glucopyranoside is used as the internal standard.
The analysis is carried out using the following equipment:

- A gas chromatograph (type VARIAN 3400) equipped with:

  ◦ A split-splitless injector fitted with a split liner,
  ◦ A flame ionisation detector,
  ◦ An integrator-recorder or any computer system for processing the detector signal.

- A DB-1 GC capillary column

  ◦ Length: 40 metres

◦ Internal diameter: 0.18 millimetres
◦ Film thickness: 0.40 microns

Chromatographic conditions:

- Column temperature: 100°C -- 3°C/min $\rightarrow$ 260°C -- 10°C/min $\rightarrow$ 300°C - Hold 12.66 min
- Injector temperature (split/splitless): 300°C
- FID detector temperature: 300°C
- Carrier gas: Helium
- Injection mode: split
- Split ratio: 40
- Pressure mode: constant flow - flow = 1.00 mL/min *(44 psi initial)*
- Make up (nitrogen): 25 mL/min
- Air: 300ml/min - $H_2$: 30 mL/min
- Injected volume: 1$\mu$L

According to the following protocol:

1) Internal standard solution
Prepare a solution of méthylalpha-D-glucopyranoside at approximately precisely 0.3 mg/mL in osmosis water.
2) Preparing the derivative
In a tare box, weigh approximately, precisely 100 to 200mg of sample, add 10 mL of the internal standard solution. Leave under magnetic stirring until completely dissolved. In a 2 mL tube, place 1 mL of the solution, evaporate to dryness under a stream of nitrogen. Add approximately 20mg of methoxylamine hydrochloride. Add 1 mL of pyridine. Cap and hold for 40 minutes at 70°C in Reacti-Therm. Add 0.5 mL of BSTFA and maintain 30 min at 70°C in Reacti-Therm.
3) Calculation and system calibration are carried out in the same way as for total sugars.

***Total and free acid sugars composition:***

Throughout the present application, the contents of free uronic acids or total uronic acids are determined using HPAEC-PAD.
Free and total uronic acids methods are that described in the publication of 2004, Journal of Agricultural and Food Chemistry 4652 "New Method for a Two-Step hydrolysis and chromatographic analysis of pectin neutral sugar chains" with some adaptations. The chemical hydrolysis with 5 mL of 0.2 M TFA is carried out at 80°C for 6 hours instead of 72 hours. For the analysis of free uronic acids, the sample is diluted in water. After homogenization, the sample is filtered through a ww PTFE 0,45 micron filter and injected. Here, the free and total uronic acids are assimilated to free and total galacturonic acid.

***Bound neutral and acidic sugars composition:***

For the following structural characterization, it must be considered only the pectic saccharides bounded in the pectic structure, without monosaccharides that can be present in the product composition.
For each (neutral or acid) sugar, the bound sugar content is determined by subtracting the free sugar content from the total sugar content. For e.g., the mass content of bound galactose is expressed as the subtraction of the mass content of free galactose from the mass content of total galactose. This method of calculation is similar for galacturonic acid, rhamnose, arabinose, mannose, xylose, fucose and ribose.
Due to the eventual presence of starch in product composition, the calculation must be adapted for the glucose. The mass content of bound glucose is calculated by subtracting the mass content of glucose from starch and the mass content of free glucose from the mass content of total glucose.
The mass glucose content from starch is expressed as percentage the mass content of starch divided by the factor 0.9.

***Homogalacturonan (HG) and rhamnogalacturonan-I (RG-I) composition:***

The composition in chains HG and chains RG-I, expressed as molar, is determined according to methods described by (M'sakni, N., & al, European Polymer Journal, 2006, 42) and by (Yang, J.-S., & et al., Food Chemistry, 2018, 244) with some calculations adaptation.
For characterization of structural pectic polysaccharides, the molar content of bound sugars must be expressed by considering only the pectic saccharides, without other compounds (like proteins, starch, ashes...).

In this restricted matrix, the molar content of each bound (neutral and acid) sugar is expressed as percentage as the molar content of corresponding bound sugar divided by the sum of molar contents of all bound sugars (i.e., galacturonic acid, galactose, glucose, rhamnose, arabinose, mannose, xylose, fucose, and ribose).

The molar amount of each bound sugar is determined from the corresponding mass amount and the molar weight. For e.g., the molar content of bound galactose is expressed as the mass content of bound galactose divided by the molar weight of galactose (i.e., 180.16 g/mol).

According to these molar contents, the homogalacturonan (HG) and rhamnogalacturonan-I (RG-I) composition is determined by these formulas:

- The molar content in chains HG is: HG (mol%) = GalA (mol%) - Rha (mol%)
- The molar content is chains RG-I is: RG-I (mol%) = [GalA (mol%) - HG (mol%)] + Rha (mol%) + Gal (mol%) + Ara (mol%)
- The molar GalA to Rha ratio is: GalA (mol%) / Rha (mol%)
- The molar (Gal plus Ara) to GalA ratio is: [Gal (mol%) + Ara (mol%)] / GalA (mol%)
- The molar Ara to Rha ratio is: Ara (mol%) / Rha (mol%)
- The molar GalA to Gal ratio is: GalA (mol%) / Gal (mol%)

**Quantification of total and free methanol and total and free acetate:**

Quantification of total and free methanol and total and free acetate was carried out by proton NMR at 60°C in D2O, using an internal standard (Na benzoate ≈ 20 mg on dry basis / g).

The samples were run twice, the first time in solution in D2O and the second time with NaOD added. The purpose of the sodium hydroxide is to saponify the sample and release the methyl and acetyl compounds bound to it. The difference between the total acetate level in the saponified sample and the free acetate level in the crude sample gives us the previously fixed acetate level. The reasoning is the same for the methyl function.

The procedure is as follows:

- Weigh approximately 10 mg of sample into the NMR tube and add an ampoule of deuterium oxide (0.75 mL), then add approximately 30 mg of internal standard solution into the tube. Stir until dissolved by placing it in an ultrasonic bath.
- Fit the spinner onto the tube and place it in the magnet.
- Perform the acquisition, without solvent suppression, with a relaxation time of at least 10 s, without rotation, after the appropriate instrument settings (field, lock phase and shims), at a temperature of 60°C. The spectral window must be at least between 0 and 9 ppm, with the water peak calibrated at 4.40 ppm.
- The spectrum is processed after Fourier transformation, phase correction and baseline subtraction in manual mode (without exponential multiplication, LB=GB=0).
- For the second analysis, in the same tube, add 100 μL of 2N NaOD solution. Stir, then place the tube in a water bath for a few minutes (to colour the solution). Repeat the acquisition under the same conditions as before.

Quantification of free species or species released after saponification (in g/100g on wet basis):

The free species found or released after saponification (methanol and sodium acetate) in pectic saccharides are expressed in g per 100g on wet basis. To obtain these results, integration must be carried out on the spectrum analysed in water and on the spectrum analysed with the addition of NaOD.

$$Methanol = \frac{S_{MeOH} \times P_E \times W_E \times C_E \times M_{MeOH} \times 100}{S_E \times P_{MeOH} \times M_E \times W_s}$$

$P_E$: number of protons from the internal standard ($P_E$ = 5)
$P_{MeOH}$: number of protons from methanol ($P_{MeOH}$ = 3)
$W_E$: mass, in grams, of weighed internal standard solution
$C_E$: concentration, in milligrams on dry basis / gram of Na benzoate solution
$M_E$: molar mass, in grams per mole, of Na benzoate ($M_E$ =144 g/mol)
$M_{MeOH}$ : molar mass, in grams per mole, of methanol ($M_{MeOH}$=32g/mol)
$W_s$ : mass, in milligrams, of the sample to be analysed.
$S_E$ : Surface area of Na benzoate=100
$S_{MeOH}$ : Surface area of methanol

$$Acetate = \frac{S_{Ac} \times P_E \times W_E \times C_E \times M_{Ac} \times 100}{S_E \times P_{Ac} \times M_E \times W_s}$$

$P_E$: number of protons from the internal standard ($P_E$ = 5)

$P_{Ac}$: number of protons from acetate ($P_{Ac}$ = 3)

$W_E$: mass, in grams, of weighed internal standard solution.

$C_E$: concentration, in milligrams on dry basis / gram of Na benzoate solution

$M_E$: molar mass, in grams per mole, of Na benzoate ($M_E$ =144 g/mol)

$M_{Ac}$ : molar mass, in grams per mole, of acetate ($M_{Ac}$=82g/mol)

$W_s$ : mass, in milligrams, of the sample to be analysed.

$S_E$ : Surface area of Na benzoate=100

$S_{MeOH}$ : Surface area of acetate

Quantification of fixed species (in g/100g on wet basis):

The content of bound methanol is determined by subtracting the free methanol content from the total methanol content.

Bound methanol (in % by wet weight) = Total methanol after saponification (in % by wet weight) - Free methanol (in % by wet weight)

The content of bound sodium acetate is calculated in the same way.

Bound acetate (in % by wet weight) = Total acetate after saponification (in % by wet weight) - Free acetate (in % by wet weight).

*Degrees of esterification and acetylation:*

The degree of esterification is expressed as percentage as the molar amount of bound methanol divided by the molar amount of bound galacturonic acid.

In the same way, the degree of acetylation is expressed as percentage as the molar amount of bound sodium acetate divided by the molar amount of bound galacturonic acid.

*Starch content:*

Throughout the present application, the content of starch was determined according to the following method:

- Add test sample to the glass jars with metal lids, containing around 750 mg of starch.
- Add 60 to 100 mL distilled water and homogenize.
- Adjust the pH of the aqueous suspension to be between 6-7.
- Keep the jar for 3 minutes in a boiling water bath while shaking the jar. Then transfer to the oven at 130°C for an hour leaving the jar closed.
- At the exit of the oven leave them on the bench for 10 min before cooling them in a cold water bath and depressurize the jar.
- Add 5 mL of 1,2 M Sodium Acetate solution pH 4,6 and adjust the pH à 4,6 if necessary.
- Add 500 μL of amyloglucosidase (E-AMGDF from Megazyme).
- Place the jar in a 60°C water bath for 2 hours.
- After cooling, transfer the test portion to a 500 mL volumetric flask and make up to volume with distilled water. Homogenize and filter.
- Carry out the enzymatic determination of glucose by the hexokinase method to obtain total released glucose.
- Analyze the free glucose in the sample and deduct it from the total glucose result obtained after hydrolysis.
- Calculate the amount of starch (expressed in %): glucose concentration in g/L of the sample (total released glucose - free glucose) $\times$ 0,9 (conversion factor from glucose to starch) $\times$ 0,5 (volume of volumetric flask in ml/1000) $\times$ (100/weight of wet product in g).

**Example 1. Preparation of the hydrolyzed pectic polysaccharides according to the invention**

Three samples (A1, B1 and C1) of hydrolyzed pectic polysaccharides were produced according to the method in accordance with the present invention.

Potato pulps from the potato-processing industry present the following composition.

| Analytical criteria | | For the production of Samples A1-A3 | For the production of Samples B1-B3 | For the production of Samples C1-C3 |
|---|---|---|---|---|
| Moisture (% by weight) | | 17.7 % | 19.2 % | 16.7 % |
| Starch content (% by dry weight) | | 19.4 % | 27.8 % | 31.8 % |
| Total sugars content (% by dry weight) | Galacturonic acid | 11.3 % | 10.7 % | 8.8 % |
| | Galactose | 19.3 % | 16.8 % | 17.9 % |
| | Rhamnose | 1.5 % | 1.0 % | 1.1 % |
| | Arabinose | 4.4 % | 3.5 % | 1.1 % |
| | Glucose | 29.6 % | 37.4 % | 39,0 % |
| | Mannose | 0.3 % | 0.3 % | 0,3 % |
| | Xylose | 1.8 % | 1.3 % | 1,4 % |
| | Fucose | nd | nd | nd |
| | Ribose | nd | nd | nd |
| Note: nd for undetectable and/or unquantifiable | | | | |

To produce the Sample A1, 115 kg of potato pulp is loaded in the "ploughshare mixer type DVT-300/1 MZ" from the supplier Lödige and under stirring, demineralized water is added in the mixer to get a dry substance of 10 %.
Still under stirring, the pectin-rich substrate is pre-heated until 50°C.
When the temperature is reached, the enzymatic treatment is started by adding the pectinase (ISOZM PG200) in the mixer at a dose of 30 uPG by grams of pulp on dry matter.
The pH of pectin-rich substrate is controlled in beginning of the enzymatic hydrolysis, a pH value of 5.2 is obtained.
Then the reaction is carried out for 2h still under stirring and maintaining the temperature at 50°C. When the treatment time is up, the hydrolyzed pulp feeds, in batch mode, a "hydraulic filter press type HPX207" from the supplier Bucher Unipektin.
The machine parameters are adjusted so that:

- the insoluble residue pulp has a dry substance of 26 % and,
- the filtrate has an insoluble content below 2 % v/v (lab centrifuge spin test performed at 5000 g for 5 min), and,
- the dry substance yield for the filtrate is 27 % in weight.

The total filtration time gets 150 min.
As soon as the filtration is finished, the filtrate is loaded in a double jacket tank, and heats at 80°C for 30 min in order to inactivate the pectinase.
Then the hydrolyzed pectic polysaccharides are recovered, freeze-dried, and labelled Sample A1.
To produce the Sample B1, 350 kg of potato pulp is loaded in a double jacket tank equipped with a turbine agitator, and under stirring, decarbonated water is added in the tank to get a dry substance of 6.5 %.
Still under stirring, the pectin-rich substrate is pre-heated until 50°C. When the temperature is reached, the enzymatic treatment is started by adding the pectinase (ISOZM PG200) in the mixer at a dose of 25 uPG by grams of pulp on dry matter.
The pH of pectin-rich substrate is controlled in beginning of the enzymatic hydrolysis, a pH value of 5.3 is obtained.
Then the reaction is carried out for 2 h still under stirring and maintaining the temperature at 50°C. When the treatment time is up, the hydrolyzed pulp feeds in continuous a "rotary vacuum filter type precoat" from the supplier NIVOBA with 2 m$^2$ filtration surface.

Beforehand, the precoat layer is formed on filter with 100 kg of potato starch.

The machine parameters are adjusted so that:

- the insoluble residue pulp has a dry substance of 17 % and,
- the filtrate has an insoluble content below 2 % v/v (lab centrifuge spin test performed at 5000 g for 5 min), and,
- the dry substance yield for the filtrate is 22 % in weight.

The total filtration time gets 120 min.

As soon as the filtration is finished, the filtrate feds a UHT skid at 130°C in continuous for contact time of 5min in order to inactivate the pectinase.

Then the hydrolyzed pectic polysaccharides are recovered, freeze-dried, and labelled Sample B1.

To produce the Sample C1, 300 kg of potato pulp is loaded in a double jacket tank equipped with a turbine agitator, and under stirring, decarbonated water is added in the tank to get a dry substance of 5.5 %.

Still under stirring, the pectin-rich substrate is pre-heated until 50°C.

The pH of pectin-rich substrate is controlled and is adjusted at 3.5 by adding hydrochloric acid solution at concentration of 5 % in weight.

When the temperature is reached, the enzymatic treatment is started by adding the pectinase (ISOZM PG200) in the mixer at a dose of 50 uPG by grams of pulp on dry matter.

Then the reaction is carried out for 3 h still under stirring and maintaining the temperature at 50°C. When the treatment time is up, the hydrolyzed pulp feeds in continuous a "centrifuge decanter type Z2" from the supplier Flottweg.

The machine parameters are adjusted so that:

- the insoluble residue pulp has a dry substance of 18 % and,
- the filtrate has an insoluble content below 2 % v/v (lab centrifuge spin test performed at 5000 g for 5 min), and,
- the dry substance yield for the filtrate is 25 % in weight.

The total filtration time gets 50 min.

As soon as the filtration is finished, the overflow is loaded in a double jacket tank, and heats at 80°C for 30 min in order to inactivate the pectinase.

Then the hydrolyzed pectic polysaccharides are recovered, freeze-dried, and labelled Sample C1.

### Example 2. Preparation of the high molecular weight pectic saccharides according to the invention

Three samples (A2, B2 and C2) of high molecular weight saccharides were produced according to the method in accordance with the present invention.

To produce the Sample A2, the same experimental procedure is followed than the production of Sample A1, until the pectinase inactivation step included.

From this point, the heat-treated filtrate which contains the hydrolyzed pectic polysaccharides is cooled until 45°C in double jacket tank and then feeds a "rotary dynamic cross-flow filter type DYNAMOS 1" from the supplier TMCI PADOVAN.

The machine is equipped with 8 ceramic discs of molecular cut-off of 0.5 $\mu$m from the supplier TMCI PADOVAN.

The microfiltration is operated in batch mode, by recycling the retentate in the feed tank and by going out the permeate.

The machine parameters are adjusted so that:

- the filtration temperature is controlled at 45°C and,
- the transmembrane pressure is regulated at 1.1 bar and,
- the average dry substance of permeate is 0.8 % and,
- the yield for the retentate is 34 % in weight.

Then the retentate which contains the high molecular weight pectic saccharides is recovered, spray-dried, and labelled Sample A2.

To produce the Sample B2, the same experimental procedure is followed than the production of Sample B1, until the pectinase inactivation step included.

From this point, the heat-treated filtrate is cooled until 65°C in double jacket tank and then feeds a "tangential microfiltration type SIVALAB" from the supplier SIVA.

The machine is equipped with two carters of a tubular ceramic membrane from the supplier TAMI Industries.

The membrane characteristics are: INSIDE CéRAM series, molecular cut-off of 0.45 $\mu$m, 23 channels,

membrane diameter of 25mm, membrane length of 1178 mm.

The microfiltration is operated in batch mode, by recycling the retentate in the feed tank and by going out the permeate.

The machine parameters are adjusted so that:

- the filtration temperature is controlled at 65°C and,
- the transmembrane pressure is regulated at 1.6 bar and,
- the average dry substance of permeate is 2.3 % and,
- the yield for the retentate is 14 % in weight.

Then the retentate which contains the high molecular weight pectic saccharides is recovered, spray-dried, and labelled Sample B2.

To produce the Sample C2, the same experimental procedure is followed than the production of Sample C1, until the pectinase inactivation step included.

From this point, the heat-treated filtrate is cooled until 60°C in double jacket tank and then feeds a "tangential microfiltration type 12-E-042" from the supplier TIA ("Techniques Industrielles Appliquées", France).

The machine is equipped with one carter of 7 tubular ceramic membranes from supplier TAMI Industries.

The membrane characteristics are: INSIDE CéRAM series, molecular cut-off of 0.8 $\mu$m, 8 channels, membrane diameter of 25 mm, membrane length of 1020 mm.

The microfiltration is operated in batch mode, by recycling the retentate in the feed tank and by going out the permeate.

The permeate is loaded in a double jacket tank and maintained at a temperature of 60°C.

The machine parameters are adjusted so that:

- the filtration temperature is controlled at 60°C and,
- the transmembrane pressure is regulated at 1.0 bar and,
- the average dry substance of permeate is 1.3 % and,
- the yield for the retentate is 20 % in weight.

Then the retentate from microfiltration of 0.8 $\mu$m is recovered.

The permeate from microfiltration of 0.8 $\mu$m feeds a "tangential microfiltration type SIVALAB" from the supplier SIVA.

The machine is equipped with two carters of a tubular ceramic membrane from the supplier TAMI Industries.

The membrane characteristics are: INSIDE CéRAM series, molecular cut-off of 0.45 $\mu$m, 23 channels, membrane diameter of 25 mm, membrane length of 1178 mm.

The microfiltration is operated in batch mode, by recycling the retentate in the feed tank and by going out the permeate.

The machine parameters are adjusted so that:

- the filtration temperature is controlled at 60°C and,
- the transmembrane pressure is regulated at 2.0 bar and,
- the average dry substance of permeate is 1.3 % and,
- the yield for the retentate is 5 % in weight.

Then the retentate from microfiltration of 0.45 $\mu$m is recovered and mixed with the retentate from microfiltration of 0.8 $\mu$m. The retentate mix which contains the high molecular weight pectic saccharides is spray-dried, and labelled Sample C2.

### Example 3. Preparation of the low molecular weight pectic saccharides according to the invention

To produce the Sample A3, the same experimental procedure is followed than the production of Sample A2, until the microfiltration step included with membrane cut-off of 0.5 $\mu$m.

From this point, the permeate is recovered, loaded in a double jacket tank and cooled at a temperature of 40°C.

Then, the permeate feeds a "tangential nanofiltration type 23-E-028" from the supplier TIA ("Techniques Industrielles Appliquées", France). The machine is equipped with one carter of one organic spiral wounded membrane from supplier SUEZ.

The membrane characteristics are: DL series, molecular cut-off of 150-300 Da, membrane diameter of 40.0 inches, spacer mil of 50mm, membrane reference "DL2540C50".

The nanofiltration is operated in batch mode, by recycling the retentate in the feed tank and by going out the permeate.

The machine parameters are adjusted so that:

- the filtration temperature is controlled at 40°C and,
- the transmembrane pressure is regulated at 30 bar and,
- the average dry substance of retentate is 10.8 % and,
- the yield for the retentate is 13 % in weight.

Then the retentate from nanofiltration which contains the low molecular weight pectic saccharides is recovered, spray-dried, and labelled Sample A3.

To produce the Sample B3, the same experimental procedure is followed than the production of Sample B2, until the microfiltration step included with membrane cut-off of 0.45μm.

From this point, the permeate is recovered, loaded in a double jacket tank and cooled at a temperature of 35°C.

Then, the permeate feeds a "tangential nanofiltration type 23-E-028" from the supplier TIA ("Techniques Industrielles Appliquées", France).

The machine is equipped with one carter of one organic spiral wounded membrane from supplier SUEZ.

The membrane characteristics are: DL series, molecular cut-off of 150-300 Da, membrane diameter of 40.0 inches, spacer mil of 50mm, membrane reference "DL2540C50".

The nanofiltration is operated in batch mode, by recycling the retentate in the feed tank and by going out the permeate.

The machine parameters are adjusted so that:

- the filtration temperature is controlled at 35°C and,
- the transmembrane pressure is regulated at 30 bar and,
- the average dry substance of retentate is 8.9% and,
- the yield for the retentate is 15% in weight.

Then the retentate from nanofiltration which contains the low molecular weight pectic saccharides is recovered, spray-dried, and labelled Sample B3.

To produce the Sample C3, the same experimental procedure is followed than the production of Sample C2, until the microfiltration step included with membrane cut-off of 0.45μm.

From this point, the permeate is recovered, loaded in a double jacket tank and cooled at a temperature of 35°C.

Then, the permeate feeds a "tangential nanofiltration type 23-E-028" from the supplier TIA ("Techniques Industrielles Appliquées", France).

The machine is equipped with one carter of one organic spiral wounded membrane from supplier SUEZ.

The membrane characteristics are: DL series, molecular cut-off of 150-300 Da, membrane diameter of 40.0 inches, spacer mil of 50mm, membrane reference "DL2540C50".

The nanofiltration is operated in batch mode, by recycling the retentate in the feed tank and by going out the permeate.

The machine parameters are adjusted so that:

- the filtration temperature is controlled at 50°C and,
- the transmembrane pressure is regulated at 30 bar and,
- the average dry substance of retentate is 24.0% and,
- the yield for the retentate is 6% in weight.

Then the retentate from nanofiltration which contains the low molecular weight pectic saccharides is recovered, spray-dried, and labelled Sample C3.

**Example 4. Characterization of the high or low molecular weight pectic saccharides according to the invention**

The chemical structures of the hydrolyzed pectic polysaccharides, the low molecular weight and high molecular weight pectic saccharides following the invention are given is the following table.

For comparison purpose, are given the chemical structures of the following polysaccharides:

- SOYAFIBE-S-DN (two samples), SOYAFIBE-S-CA300 and SOYAFIBE-S-DA100, as water-soluble soybean hemicellulose, from the company Fuji Oil, and produced from okara (soybean pulp residue)

- GENU Pectin PAN as high ester pectin, from the company CP Kelco, and produced from sugar beet pulp residue.
- Rhamnogalacturonan I (Potato) of reference P-RHAM1 and Galactan (Potato) of reference P-GALPOT, analytical standards used in research, from the company MEGAZYME, and produced from potato fiber.

| | HG (mol%) | Methylation degree (mol%) | Acetylation degree (mol%) | RG-I (mol%) | GalA/ Rha (mol/mol) | (Gal+Ara) / GalA (mol/mol I) | (Gal+Ara ) / Rha (mol/mol ) | Gal / Rha (mol/mol I) | Ara/ Rha (mol/ mol) | GalA/Gal (mol/mol) |
|---|---|---|---|---|---|---|---|---|---|---|
| Hydrolyzed pectic polysaccharides (samples A1, B1, C1) | 22 - 27 | 44 - 57 | 48 | 71 74 | 6.6 - 7.4 | 2.0 - 2.6 | 13 - 19 | 10 - 16 | 2.8 3.4 | 0.5 - 0.6 |
| Low molecular weight pectic saccharides (samples A3, B3, C3) | 22 - 33 | 23 - 44 | 11 - 38 | 66 74 | 6.8 - 26 | 1.8 - 2.6 | 18 - 55 | 15 - 48 | 3.1 6.4 | 0.5 - 0.6 |
| High molecular weight pectic saccharides (samples A2, B2, C2) | 16-25 | 17 - 39 | 51 - 90 | 73 83 | 4.1 - 5.5 | 1.9 - 3.5 | 10 - 14 | 7 - 11 | 2.4 3.5 | 0.4-0.7 |
| SOYAFIBE-SDN | 11 - 12 | 5.7 - 9.9 | ≤ 0.6 | 77 79 | 4.3 - 4.7 | 4.4 - 5.2 | 21 - 22 | 13 - 14 | 7.6 8.2 | 0.3-0.4 |
| SOYAFIBE-S-CA300 | 13 | 8.4 | nd | 75 | 5.5 | 4.5 | 25 | 16 | 9.0 | 0.4 |
| SOYAFIBE-S-DA100 | 13 | 8.6 | 1.6 | 77 | 4.8 | 4.5 | 22 | 14 | 7.9 | 0.4 |
| GENU Pectin PAN | 64 | 58 | 21 | 34 | 17 | 0.4 | 6.5 | 4.2 | 2.3 | 4.1 |
| P-RHAM1 | 64 | 29 | 24 | 35 | 14 | 0.4 | 5.2 | 4.6 | 0.6 | 3.1 |
| P-GALPOT | 8 | nd | nd | 91 | 3.2 | 7.7 | 25 | 24 | 1.2 | 0.1 |
| Note: nd for undetectable | | | | | | | | | | |

The hydrolyzed pectic polysaccharides, the low molecular weight and high molecular weight pectic saccharides following the invention have a high content in rhamnogalacturonan-I chains and a low content in homogalacturonan chains.

Comparatively to comparative polysaccharides, the hydrolyzed pectic polysaccharides and the high molecular weight pectic saccharides have simultaneously a higher methylation degree and higher acetylation degree.

Both present simultaneously an intermediary range of ratios for GalA / Rha, (Gal+Ara) / GalA and Gal / Rha.

The low molecular weight pectic saccharides have a particular intermediary range of methylation degree and acetylation degree. In comparison to all (poly)-saccharides, the low molecular weight pectic saccharides present simultaneously very high ratios for GalA / Rha, (Gal+Ara) / GalA and Gal / Rha.

The composition and molecular weights of the hydrolyzed pectin rich substrate, the low molecular weight and high molecular weight pectic saccharides following the invention are given is the following tables.

*Molecular weights (Da)*

|  | Average molecular weight Mw (equivalent to Pullulan) | Fraction lower than 1500 Da | Fraction from 1500 Da to 100 kDa | Fraction higher than 100 kDa |
|---|---|---|---|---|
| Hydrolyzed pectic polysaccharides (samples A1, B1, C1) | ≥ 100,000 Da | 16 - 19% | 52 - 58% | 26 - 29% |
| Low molecular weight pectic saccharides (samples (A3, B3, C3) | 9,100 - 81,000 | 17 - 25% | 65 - 79% | 1 - 17% |
| High molecular weight pectic saccharides (samples (A2, B2, C2) | ≥ 100,000 Da | 7 - 12% | 47 - 56% | 32 - 46% |
| SOYAFIBE-S-DN | ≥ 100,000 Da | 8% | 31 - 32% | 60 - 61% |
| SOYAFIBE-S-CA300 | > 100,000 Da | 6% | 28% | 66% |
| SOYAFIBE-S-DA100 | ≥ 100,000 Da | 8% | 30% | 62% |
| GENU Pectin PAN | ≥ 100,000 Da | 4% | 25% | 71% |
| P-RHAM1 | ≥ 100,000 Da | 2% | 59% | 38% |
| P-GALPOT | ≥ 100,000 Da | 0% | 62% | 38% |

The low molecular weight pectic saccharides have a particular average molecular weight (Mw), lower than the hydrolyzed pectin rich substrate, the high molecular weight pectic saccharides and also the comparative polysaccharides. The fraction of saccharides having a molecular weight higher than 100 kDa is lower in comparison to all (poly)-saccharides.

Comparatively to all (poly)-saccharides, the hydrolyzed pectin rich substrate presents simultaneously an intermediary range of values for the fraction lower than 1500 Da and the fraction higher than 100 kDa.

The high molecular weight pectic saccharides are simultaneously poor in saccharides having a molecular lower than 1500 Da, and have an intermediary range of values for the fraction between 1500 Da and 100 kDa, and for the fraction higher than 100 kDa.

*Total sugars composition* (*% by dry weight*)

| | Rhamnose | Fucose | Ribose | Mannose | Arabinose | Xylose | Galactose | Glucose | Galacturonic acid |
|---|---|---|---|---|---|---|---|---|---|
| Hydrolyzed pectic polysaccharides (samples A1, B1, C1) | 2.1 - 3.2 | ≤ 0.1 | < 0.1 | 0.3 - 0.4 | 7.5 - 8.4 | 0.5 - 0.9 | 37.1 - 49.7 | 2.3-8.3 | 22.0 - 27.6 |
| Low molecular weight pectic saccharides (samples A3, B3, C3) | 0.9 - 2.8 | < 0.1 | nd | 0.1 - 0.3 | 4.8 - 8.1 | 0.2-0.7 | 46.0 - 49.4 | 0.7 - 3.2 | 26.3 - 33.2 |
| High molecular weight pectic saccharides (samples A2, B2, C2) | 3.7 - 4.5 | < 0.05 | ≤ 0.1 | 0.3 - 0.4 | 9.4 - 11.8 | 0.4-0.5 | 36.2 - 44.3 | 0.9-2.9 | 17.8 - 27.6 |
| SOYAFIBE-S-DN | 2.6 - 2.9 | 2.2-2.4 | nd | 0.1 - 0.4 | 19.6 - 20.5 | 4.8 - 50 | 40.9 - 41.8 | 2.6-3.5 | 13.5 - 16.3 |
| SOYAFIBE-S-CA300 | 2.4 | 2.4 | nd | 0.1 | 19.5 | 4.9 | 41.0 | 2.8 | 15.5 |
| SOYAFIBE-S-DA100 | 2.8 | 2.4 | nd | 0.3 | 20.4 | 4.9 | 42.4 | 2.9 | 16.1 |
| GENU Pectin PAN | 2.6 | nd | nd | 0.1 | 5.5 | 0.2 | 12.0 | 1.4 | 53.3 |
| P-RHAM1 | 2.2 | < 0.05 | < 0.05 | 0.5 | 1.2 | 0.4 | 11.2 | 0.4 | 37.2 |
| P-GALPOT | 2.3 | < 0.05 | < 0.05 | < 0.05 | 2.5 | 0.5 | 60.0 | 0.3 | 8.8 |
| Note: nd for undetectable | | | | | | | | | |

The hydrolyzed pectic polysaccharides, the low molecular weight and high molecular weight pectic saccharides following the invention are simultaneously rich in galactose and in galacturonic acid, and poor in fucose, in rhamnose, in xylose and in arabinose.

Comparatively to hydrolyzed pectic polysaccharides and the high molecular weight pectic polysaccharides, the low molecular weight pectic saccharides are richer in galactose and in galacturonic acid, and poorer in arabinose and rhamnose.

*Free sugars composition* (*% by dry weight*)

| | Rhamnose | Fucose | Ribose | Mannose | Arabinose | Xylose | Galactose | Glucose | Galacturonic acid |
|---|---|---|---|---|---|---|---|---|---|
| Hydrolyzed pectic polysaccharides (samples A1, B1, C1) | < 0.05 | nd | nd | < 0.05 | $\leq 0.2$ | < 0.05 | $\leq 0.6$ | $\leq 1.9$ | 0.1 - 2.2 |
| Low molecular weight pectic saccharides (samples A3, B3, C3) | < 0.05 | nd | nd | < 0.05 | $\leq 0.2$ | < 0.05 | $\leq 1.1$ | $\leq 0.3$ | 0.6 - 4.8 |
| High molecular weight pectic saccharides (samples A2, B2, C2) | < 0.05 | nd | nd | < 0.05 | $\leq 0.1$ | < 0.05 | $\leq 0.6$ | < 0.1 | < 2.1 |
| SOYAFIBE-S-DN | nd | nd | nd | nd | <0.1 | nd | <0.1 | $\leq 0.3$ | <0.1 |
| SOYAFIBE-S-CA300 | nd | nd | nd | nd | nd | nd | nd | 0.1 | <0.1 |
| SOYAFIBE-S-DA100 | nd | nd | nd | nd | nd | nd | nd | 0.2 | <0.1 |
| GENU Pectin PAN | nd | nd | nd | nd | nd | nd | nd | <0.1 | <0.1 |
| P-RHAM1 | nd | nd | nd | nd | nd | nd | nd | nd | <0.1 |
| P-GALPOT | nd | nd | nd | nd | nd | nd | nd | nd | <0.1 |
| Note: nd for undetectable | | | | | | | | | |

The hydrolyzed pectic polysaccharides and the high molecular weight pectic polysaccharides have very low content in monosaccharides.

Only, the low molecular weight pectic polysaccharides have quantifiable content in monosaccharide of galacturonic acid.

## Claims

1. Method of producing high and low molecular weight pectic saccharides that are enriched in homogalacturonan (HG) and rhamnogalacturonan-I (RG-I), said method comprising the steps of:

   - providing potato pulp as pectin-rich substrate,
   - subjecting the pectin-rich substrate to enzymatic treatment, said enzymatic treatment comprising the use of one pectinase having an endo-polygalacturonase (EC 3.2.1.15) activity,
   - subjecting the hydrolyzed pectic polysaccharides to microfiltration using microfiltration membranes having a molecular cut-off of 0.45 and 0.8 $\mu$m,
   - recovering the microfiltration retentate,
   - subjecting the microfiltration permeate to nanofiltration using a membrane having a molecular weight cut-off in the range of 150 - 300 Da,
   - recovering the nanofiltration retentate.

2. Method of claim 1, **characterized in that** the hydrolyzed pectic polysaccharides have:

   - a molecular weight distribution for the fractions:

     ◦ lower than 1500 Da, of between 10 and 25 %, preferably between 16 and 19 %,
     ◦ from 1500 Da to 100 kDa, of between 45 and 65 %, preferably between 52 and 58 %, and
     ◦ higher than 100 kDa between 20 and 35%, preferably between 26 and 29%,

   - between 20 and 30 % in mol%, preferably between 22 and 27 % in mol% of HG,
   - between 70 and 75 % in mol% preferably between 71 and 74 % in mol% of RG-I having a GalA to Rha ratio between 6.5 and 7.5 mol/mol, preferably between 6.6 and 7.4 mol/mol, and a (Gal plus Ara) to GalA ratio between 1.8 to 3.0 mol/mol, preferably between 2.0 and 2.6 mol/mol,
   - a methylation degree between 40 and 60 % in mol%, preferably between 44 and 57 % in mol% and an acetylation degree between 45 and 50 % in mol%, preferably between 47 and 49 % in mol%.

3. Method of claim 1 or 2, **characterized in that** the RG-I side chains of the hydrolyzed pectic polysaccharides have:

   - a (Gal plus Ara) to Rha ratio between 10 and 20 mol/mol, preferably between 13 and 19 mol/mol,
   - a Gal to Rha ratio between 8 and 20 mol/mol, more preferably between 10 and 16 mol/mol,
   - an Ara to Rha ratio between 2.5 and 3.5 mol/mol, more preferably between 2.8 and 3.4 mol/mol,
   - a GalA to Gal ratio between 0.3 and 1.0 mol/mol, preferably between 0.5 and 0.6 mol/mol.

4. Method of claims 1 to 3, **characterized in that** the microfiltration retentate contains high molecular weight pectic saccharides having:

   - a molecular weight distribution for the fractions:

     ◦ lower than 1500 Da, of between 5 and 15 %, preferably between 7 and 12 %,
     ◦ from 1500 Da to 100 kDa, of between 40 and 65 %, preferably between 47 and 56 %, and
     ◦ higher than 100 kDa, of between 25 and 50%, preferably between 32 and 46%,

   - between 15 and 28 % in mol%, preferably between 16 and 25 % in mol% of HG,
   - between 70 and 85 % in mol%, preferably between 73 and 83 % in mol% of RG-I having a GalA to Rha ratio between 4.0 and 6.0 mol/mol, preferably between 4.1 and 5.5 mol/mol, and a (Gal plus Ara) to Gal A ratio between 1.5 to 4.0 mol/mol, preferably between 1.9 and 3.5 mol/mol,
   - a methylation degree between 15 and 40 % in mol% preferably between 17 and 39 % in mol% and an acetylation degree between 50 and 95 % in mol%, preferably between 51 and 90 % in mol%.

5. Method of claims 1 to 4, **characterized in that** the RG-I side chains of the high molecular weight pectic saccharides have:

- a (Gal plus Ara) to Rha ratio between 9 and 15 mol/mol, preferably between 10 and 14 mol/mol,
- a Gal to Rha ratio between 5 and 13 mol/mol, more preferably between 7 and 11 mol/mol,
- an Ara to Rha ratio between 2.0 and 4.0 mol/mol, more preferably between 2.4 and 3.5 mol/mol,
- a GalA to Gal ratio between 0.2 and 1.0 mol/mol, preferably between 0.4 and 0.7 mol/mol.

6. Method of claims 1 to 5, **characterized in that** the total sugar composition of the high molecular weight pectic saccharides contain:

- Ara: between 8 and 14 %w/w, preferably 9 and 12 %w/w,
- Rha: between 3.0 and 5.0 %w/w, preferably between 3.7 and 4.5 %w/w,
- Gal: between 32 and 48 %w/w, more preferably between 36 and 44 %w/w,
- GalA: between 16 and 30 %w/w, more preferably between 18 and 28 %w/w.

7. Method of claims 1 to 6, **characterized in that** the nanofiltration permeate contains low molecular weight pectic saccharides having:

- a molecular weight distribution for the fractions:

  ◦ lower than 1500 Da between 10 and 30%, preferably between 17 and 25%
  ◦ from 1500 Da to 100 kDa between 60 and 80%, preferably between 65 and 79%, and
  ◦ higher than 100 kDa between 0 and 25%, preferably between 1 and 17%,

- between 20 to 35 % in mol%, preferably between 22 to 33 % in mol% of HG
- between 65 to 75 % in mol%, preferably between 66 to 74 % in mol% of RG-I having a GalA to Rha ratio between 5 to 30 mol/mol, preferably between 7 to 26 mol/mol, and a (Gal plus Ara) to GalA ratio between 1.5 to 3.0 mol/mol, preferably between 1.8 to 2.6 mol/mol.
- a methylation degree between 20 and 35 % in mol%, preferably between 23 to 44 % in mol% and an acetylation degree between 10 to 40 % in mol%, preferably between 11 to 38 % in mol%.

8. Method of claims 1 to 7, **characterized in that** the RG-I side chains of the low molecular weight pectic saccharides have:

- a (Gal plus Ara) to Rha ratio between 15 and 60 mol/mol, preferably between 18 and 55 mol/mol,
- a Gal to Rha ratio between 10 and 50 mol/mol, more preferably between 15 and 48 mol/mol,
- an Ara to Rha ratio between 2.8 and 7.0 mol/mol, more preferably between 3.1 and 6.4 mol/mol,
- a GalA to Gal ratio between 0.3 and 0.7 mol/mol, preferably between 0.5 and 0.6 mol/mol.

9. Method of claim 1 to 8, **characterized in that** the total sugar composition of the low molecular weight pectic saccharides has:

- Ara: between 4.0 and 10.0 %w/w, preferably 4.8 and 8.1 %w/w,
- Rha: between 0.5 and 3.0 %w/w, preferably 0.9 and 2.8 %w/w,
- Gal: between 44 and 52 %w/w, more preferably between 46 and 50 %w/w,
- GalA: between 22 and 36 %w/w, more preferably between 26 and 34 %w/w.

10. High molecular weight pectic saccharides having:

- a molecular weight distribution for the fractions:

  ◦ lower than 1500 Da, of between 5 and 15 %, preferably between 7 and 12 %,
  ◦ from 1500 Da to 100 kDa, of between 40 and 65 %, preferably between 47 and 56 %, and
  ◦ higher than 100 kDa, of between 25 and 50%, preferably between 32 and 46%,

- between 15 and 28 % in mol%, preferably between 16 and 25 % in mol% of HG,
- between 70 and 85 % in mol%, preferably between 73 and 83 % in mol% of RG-I having a GalA to Rha ratio between 4.0 and 6.0 mol/mol, preferably between 4.1 and 5.5 mol/mol, and a (Gal plus Ara) to Gal A ratio between 1.5 to 4.0 mol/mol, preferably between 1.9 and 3.5 mol/mol,
- a methylation degree between 15 and 40 % in mol% preferably between 17 and 39 % in mol% and an acetylation degree between 50 and 95 % in mol%, preferably between 51 and 90 % in mol%.

11. High molecular weight pectic saccharides of claim 10, **characterized in that** their RG-I side chains has:

- a (Gal plus Ara) to Rha ratio between 9 and 15 mol/mol, preferably between 10 and 14 mol/mol,
- a Gal to Rha ratio between 5 and 13 mol/mol, more preferably between 7 and 11 mol/mol,
- an Ara to Rha ratio between 2.0 and 4.0 mol/mol, more preferably between 2.4 and 3.5 mol/mol,
- a GalA to Gal ratio between 0.2 and 1.0 mol/mol, preferably between 0.4 and 0.7 mol/mol.

12. High molecular weight pectic saccharides of claim 10, **characterized in that** their total sugar composition contains:

- Ara: between 8 and 14 %w/w, preferably 9 and 12 %w/w,
- Rha: between 3.0 and 5.0 %w/w, preferably between 3.7 and 4.5 %w/w,
- Gal: between 32 and 48 %w/w, more preferably between 36 and 44 %w/w,
- GalA: between 16 and 30 %w/w, more preferably between 18 and 28 %w/w.

13. Low molecular weight pectic saccharides having:

- a molecular weight distribution for the fractions:

  ◦ lower than 1500 Da between 10 and 30%, preferably between 17 and 25%
  ◦ from 1500 Da to 100 kDa between 60 and 80%, preferably between 65 and 79%, and
  ◦ higher than 100 kDa between 0 and 25%, preferably between 1 and 17%,

- between 20 to 35 % in mol%, preferably between 22 to 33 % in mol% of HG
- between 65 to 75 % in mol%, preferably between 66 to 74 % in mol% of RG-I having a GalA to Rha ratio between 5 to 30 mol/mol, preferably between 7 to 26 mol/mol, and a (Gal plus Ara) to GalA ratio between 1.5 to 3.0 mol/mol, preferably between 1.8 to 2.6 mol/mol.
- a methylation degree between 20 and 35 % in mol%, preferably between 23 to 44 % in mol% and an acetylation degree between 10 to 40 % in mol%, preferably between 11 to 38 % in mol%.

14. Low molecular weight pectic saccharides of claim 13, **characterized in that** their RG-I side chains have:

- a (Gal plus Ara) to Rha ratio between 15 and 60 mol/mol, preferably between 18 and 55 mol/mol,
- a Gal to Rha ratio between 10 and 50 mol/mol, more preferably between 15 and 48 mol/mol,
- an Ara to Rha ratio between 2.8 and 7.0 mol/mol, more preferably between 3.1 and 6.4 mol/mol,
- a GalA to Gal ratio between 0.3 and 0.7 mol/mol, preferably between 0.5 and 0.6 mol/mol.

15. Low molecular weight pectic saccharides of claim 13, **characterized in that** their total sugar composition contains:

- Ara: between 4.0 and 10.0 %w/w, preferably 4.8 and 8.1 %w/w,
- Rha: between 0.5 and 3.0 %w/w, preferably 0.9 and 2.8 %w/w,
- Gal: between 44 and 52 %w/w, more preferably between 46 and 50 %w/w,
- GalA: between 22 and 36 %w/w, more preferably between 26 and 34 %w/w.

16. Use of the high molecular weight pectic saccharides of any of claims 10 to 12, or able to be obtained according to the method of claim 1, in nutritional formulation, food product, dietary supplement, beverage or pharmaceutical product.

17. Use of the low molecular weight pectic saccharides of any of claims 13 to 15, or able to be obtained according to the method of claim 1, in nutritional formulation, food product, dietary supplement, beverage or pharmaceutical product.

Europäisches Patentamt
European Patent Office
Office européen des brevets

# EUROPEAN SEARCH REPORT

**Application Number**

EP 24 30 6182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LISE V. THOMASSEN ET AL: "Maximal release of highly bifidogenic soluble dietary fibers from industrial potato pulp by minimal enzymatic treatment", APPLIED MICROBIOLOGY AND BIOTECHNOLOGY, vol. 90, no. 3, 1 May 2011 (2011-05-01), pages 873-884, XP055124162, ISSN: 0175-7598, DOI: 10.1007/s00253-011-3092-y * fiber production and fractionation sections; figure 5; table 5 * | 1-17 | INV. C12P19/04 C12P19/14 |
| X | MUÑOZ-ALMAGRO NEREA ET AL: "Obtainment and characterisation of pectin from sunflower heads purified by membrane separation techniques", FOOD CHEMISTRY, ELSEVIER LTD, NL, vol. 318, 24 February 2020 (2020-02-24), XP086085444, ISSN: 0308-8146, DOI: 10.1016/J.FOODCHEM.2020.126476 [retrieved on 2020-02-24] * sections 2.2 and 2.3.2.2; figure s1; tables 1, S1 * | 1-17 | |
| X | QIU N X ET AL: "Apple Pectin Behavior Separated by Ultrafiltration", AGRICULTURAL SCIENCES IN CHINA,, vol. 8, no. 10, 1 October 2009 (2009-10-01), pages 1193-1202, XP026718758, ISSN: 1671-2927, DOI: 10.1016/S1671-2927(08)60329-6 [retrieved on 2009-10-01] * experimental section; figure 5; tables 2,3 * | 1-17 | |

**TECHNICAL FIELDS SEARCHED (IPC)**

C12P

-/--

The present search report has been drawn up for all claims

| Place of search | Date of completion of the search | Examiner |
|---|---|---|
| The Hague | 3 June 2025 | Blanco Parte, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 30 6182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG JIN-SHU ET AL: "Extraction, structure, and emulsifying properties of pectin from potato pulp", FOOD CHEMISTRY, vol. 244, 12 October 2017 (2017-10-12), pages 197-205, XP085277804, ISSN: 0308-8146, DOI: 10.1016/J.FOODCHEM.2017.10.059 * figure 1; tables 1,2 * | 13-15,17 | |
| A | NIU HUI ET AL: "A critical review of RG-I pectin: sources, extraction methods, structure, and applications", CRITICAL REVIEWS IN FOOD SCIENCE AND NUTRITION, vol. 64, no. 24, 28 April 2023 (2023-04-28), pages 8911-8931, XP093278904, USA ISSN: 1040-8398, DOI: 10.1080/10408398.2023.2204509 Retrieved from the Internet: URL:https://www.tandfonline.com/doi/pdf/10.1080/10408398.2023.2204509> * the whole document * | 1-17 | TECHNICAL FIELDS SEARCHED (IPC) |

The present search report has been drawn up for all claims

| Place of search | Date of completion of the search | Examiner |
|---|---|---|
| The Hague | 3 June 2025 | Blanco Parte, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015192247 A
- WO 2016132130 A
- WO 2000043424 A
- WO 2001096405 A
- WO 2007119366 A
- WO 2005003178 A
- WO 2013148282 A